(19) Europäisches Patentamt
European Patent Office
Office européen des brevets

(11) Publication number: **0 510 260 A2**

# (12) EUROPEAN PATENT APPLICATION

(21) Application number: **91122405.3**

(22) Date of filing: **31.12.91**

(51) Int. Cl.5: **C07D 471/04**, A61K 31/435, //(C07D471/04,235:00,221:00)

(30) Priority: **26.04.91 JP 122820/91**

(43) Date of publication of application: **28.10.92 Bulletin 92/44**

(84) Designated Contracting States: **AT BE CH DE DK ES FR GB GR IT LI LU NL SE**

(71) Applicant: **TOYO JOZO CO., LTD.**
**632-1, Mifuku Ohito-cho**
**Tagata-gun Shizuoka 410-23(JP)**

(72) Inventor: **Obara, Takumi**
**59-1, Odoi, Kannami-cho**
**Tagata-gun, Shizuoka 419-01(JP)**
Inventor: **Shuto, Satoshi**
**690-10, Makinokou, Shuzenji-cho**
**Tagata-gun, Shizuoka 410-24(JP)**
Inventor: **Toriya, Minoru**
**854-1, Mifuku, Ohito-cho**
**Tagata-gun, Shizuoka 410-23(JP)**
Inventor: **Fujiwara, Tatsuro**
**310, Nirayama, Nirayama-cho**
**Tagata-gun, Shizuoka 410-21(JP)**

(74) Representative: **Wächtershäuser, Günter, Dr.**
**Tal 29**
**W-8000 München 2(DE)**

## (54) 6'-C-alkyl-3-deazaneplanocin A derivative and its preparation process and use.

(57) Described herein are 6'-C-alkyl-3-deazaneplanocin A derivatives represented by the following formula:

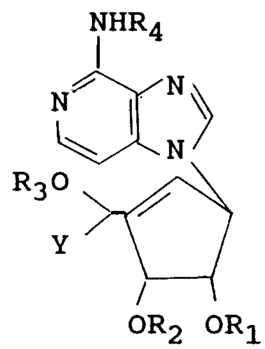

wherein Y represents a lower alkyl group, $R_1$, $R_2$ and $R_3$ individually mean a hydrogen atom or a hydroxyl-protecting group, and $R_4$ denotes a hydrogen atom or an amino-protecting group; their preparation processes; and antiviral agents containing them as an active ingredient.

EP 0 510 260 A2

## BACKGROUND OF THE INVENTION

1) Field of the Invention

This invention relates to novel derivatives of neplanocin A, and more specifically to neplanocin A derivatives useful as drugs, especially as antiviral agents and also to their preparation processes.

2) Description of the Related Art

Neplanocin A is an antibiotic having antitumor activity and growth inhibitory activity for plant pathogenic fungi, i.e., filamentous fungi. It is produced by a microorganism, *Ampullariella sp.* A11079, and is represented by the following formula (IX):

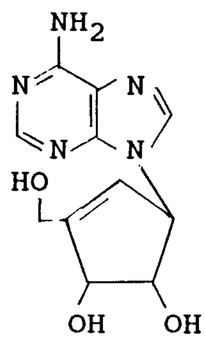

(IX)

(Japanese Patent Appln. Laid-Open No. 154792/1979). It has recently been found that this compound also has antiviral activity (ANTIMICROBIAL AGENTS AND CHEMOTHERAPY, 84-89. July 1985). Its use as an antiviral agent is therefore under investigation.

On the other hand, 3-deazaneplanocin A represented by the following formula (X):

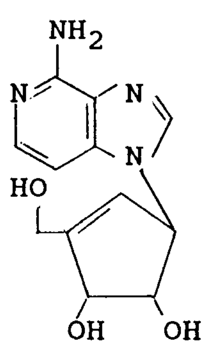

(X)

is also known as a compound having antiviral activity [J. Med. Chem. **32**, 1442-1446 (1989)].

Neplanocin A and 3-deazaneplanocin A, however, tend to have high cytotoxicity compared to their antiviral activity. This has been an unignorable problem upon their use as drugs.

There is, therefore, an outstanding demand for the development of a compound having still higher antiviral activity but low cytotoxicity.

## SUMMARY OF THE INVENTION

The present inventors have carried out an extensive investigation with a view toward overcoming the

above-described problems and obtaining a still better antiviral agent. As a result, it has been found that 6'-C-alkyl-3-deazaneplanocin As represented by the following formula (I) have good antiviral activities but low cytotoxicity, leading to the completion of the present invention.

An object of the present invention is, therefore, to provide a 6'-C-alkyl-3-deazaneplanocin A derivative represented by the following formula (I):

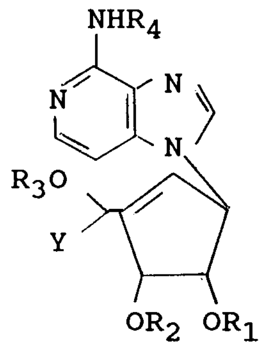

(I)

wherein Y represents a lower alkyl group, $R_1$, $R_2$ and $R_3$ individually mean a hydrogen atom or a hydroxyl-protecting group and $R_4$ denotes a hydrogen atom or an amino-protecting group, or a salt thereof.

Another object of the present invention is to provide a process for the preparation of the 6'-C-alkyl-3-deazaneplanocin A.

A further object of the present invention is to provide an antiviral agent comprising as an active ingredient the 6'-C-alkyl-3-deazaneplanocin A.

The 6'-C-alkyl-3-deazaneplanocin A derivatives according to this invention show excellent antiviral effects and, compared to neplanocin and the like, they have extremely low cytotoxicity and large chemotherapeutic indices. They can, therefore, be used advantageously as antiviral agents having high safety.

## DETAILED DESCRIPTION OF THE INVENTION AND PREFERRED EMBODIMENTS

Examples of the lower alkyl group in the 6'-C-alkyl-3-deazaneplanocin A (I) of the present invention include normal or branched alkyl groups having about 1-4 carbon atoms. Specific examples include saturated alkyl groups such as methyl, ethyl, n-propyl, i-propyl, n-butyl, i-butyl and tert-butyl; halogen-containing alkyl groups such as trifluoromethyl, difluoromethyl, monofluoromethyl and trichloromethyl; and unsaturated alkyl groups such as ethenyl and ethynyl.

The 6'-C-alkyl-3-deazaneplanocin A (I) of the present invention can be prepared, for example, in any one of the following processes.

Process 1:

Following the below-described reaction scheme, a cyclopentenone derivative represented by the formula (II) and a compound represented by the formula (III) are reacted in an inert solvent so that a compound represented by the formula (IV) is obtained. The 4-hydroxyl group is then acylated to obtain a compound represented by the formula (V). After this compound (V) is then subjected to allylic rearrangement to form a compound represented by the formula (VI), the 1-substituent group of the compound (VI) is either substituted or converted to obtain a compound represented by the formula (VII). A 3-deazaadenine derivative represented by the formula (VIII) is reacted to the compound (VII) and, if desired, one or more of the protecting groups are eliminated from the reaction product, whereby the target compound, 6'-C-alkyl-3-deazaneplanocin A (I), is obtained.

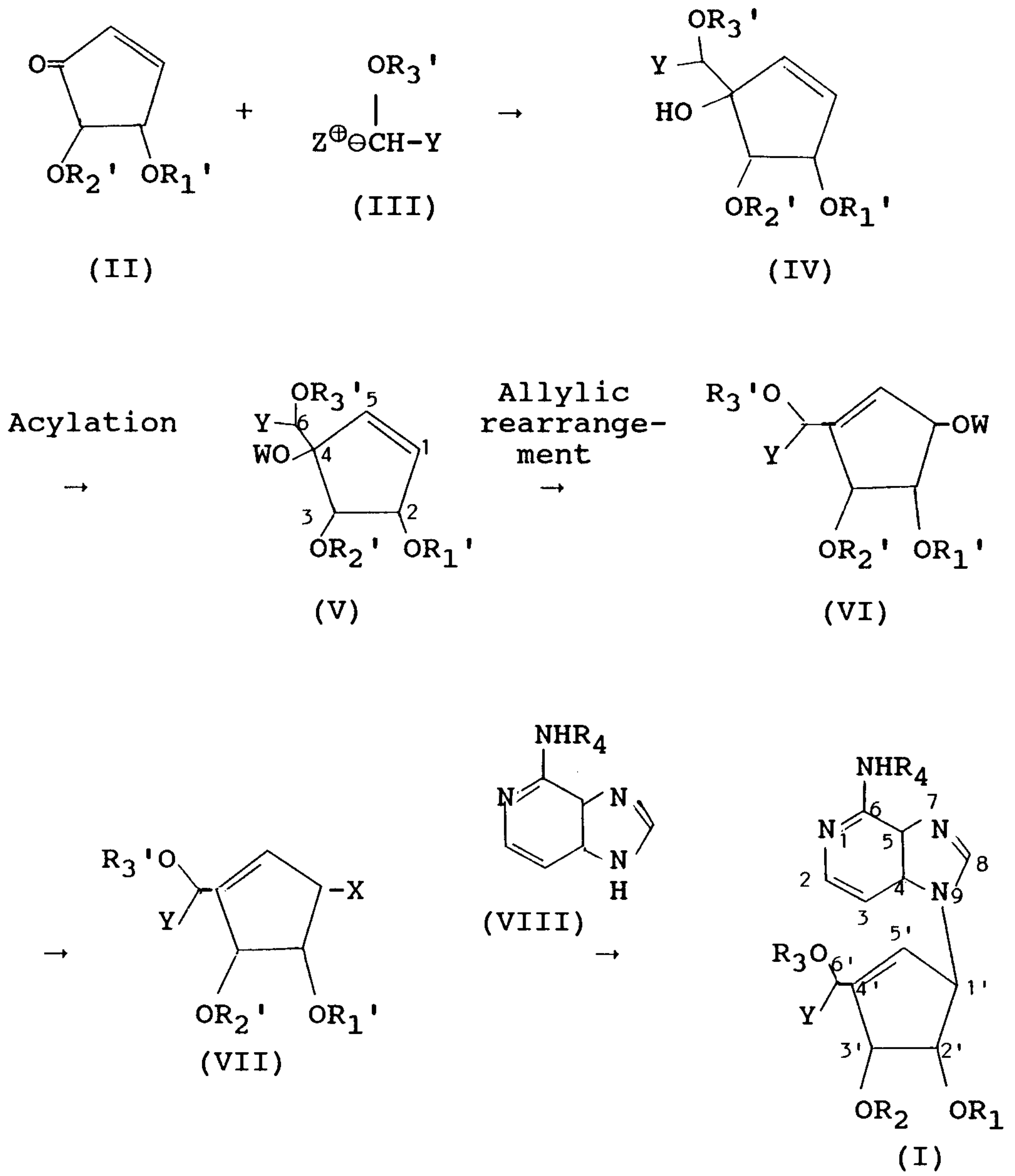


wherein Y, $R_1$, $R_2$, $R_3$ and $R_4$ have the same meanings as defined above, $R_{1'}$, $R_{2'}$ and $R_{3'}$ individually mean a hydroxyl-protecting group, X is an electron-attracting eliminative group, W represents an acyl group, and Z denotes a counter-cation for stabilizing alkyl anions.

In the above reaction scheme, no particular limitation is imposed on the hydroxyl-protecting groups $R_{1'}$-$R_{3'}$, the hydroxyl-protecting groups in $R_1$-$R_3$ and the amino-protecting group in $R_4$. In general, those employed in sugar or nucleic acid chemistry can be suitably chosen for use in the present invention. Further, the above hydroxyl-protecting groups may protect the hydroxyl groups either singly or in combination.

Particularly preferred examples of hydroxyl-protecting groups include those capable of simultaneously protecting the 2,3-diol groups of the compound (II), for example, isopropylidene and the like, and acetal-type protecting groups such as benzylidene and ethoxymethylene groups; and, in addition, silyl protecting groups such as t-butyldimethylsilyl and diisopropylmethylsilyl groups, and ether-type protecting groups such as benzyl and methoxymethyl groups.

Preferred examples of amino-protecting groups include those permitting easy introduction and deprotection, for example, acyl-protecting groups such as benzoyl and phthaloyl groups and urethane-type protecting groups such as benzyloxycarbonyl and t-butoxycarbonyl groups.

The starting material, the compound (II), can be obtained, for example, in accordance with the process described in Tetrahedron Lett. **31,** 1509-1512 (1990).

The counter-cation (Z) of the other starting material, the compound (III), is to stabilize an alkyl anion. Its examples include cations of metals having high ionization tendency. Specific examples include Li, Al, Zn, Mg, Ti and the like, with Li being especially preferred. The counter-cation may include one or more substituents, for example, alkyl groups or halogen atoms.

The compound (III) can be prepared, for example, in accordance with the following reaction scheme, namely, by reacting the aldehyde (XIV) with the alkyltin compound (XIII) in the presence of a strong base such as n-butyllithium, protecting the hydroxyl group of the resultant compound (XV) with the protecting group $R_{3'}$ to form the compound (XVI) and then reacting an alkyl metal compound such as n-butyl lithium with the compound (XVI).

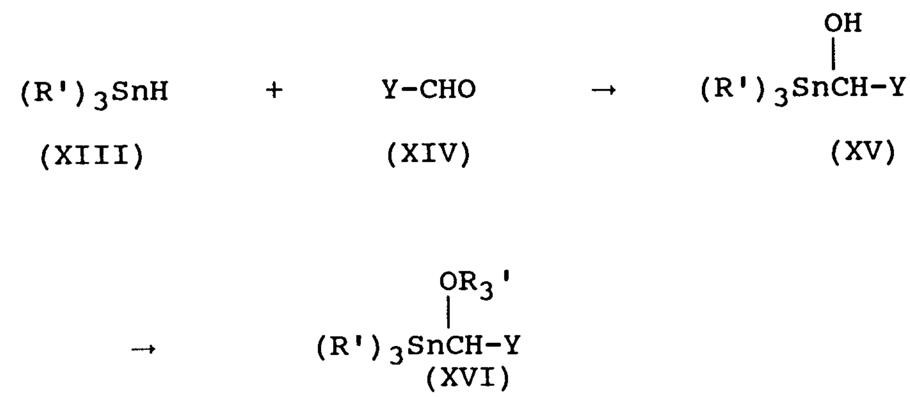

(XIII) (XIV) (XV)

(XVI)

wherein R' represents an alkyl group, and Y, $R_3$' and Z have the same meanings as defined above. Examples of the hydroxyl-protecting group $R_{3'}$ include ether-type or silyl-type hydroxyl-protecting groups such as methoxymethyl, benzyl, p-methoxybenzyl, trityl, trimethylsilyl and t-butyldimethylsilyl.

The reaction between the compound (II) and the compound (III) can be conducted in an inert solvent. Illustrative of the inert solvent include ether solvents such as tetrahydrofuran and diethyl ether. In this reaction, the compound (III) is used generally in an amount at least equal to the equivalent(s) of the compound (II), preferably 1-2 times the equivalent(s) of the compound (II).

Although no particular limitation is imposed on the reaction temperature, the reaction can be conducted generally at room temperature or lower, preferably under cooling in a dry ice-acetone system. The reaction is carried out for a period from 1 hour to overnight, in general.

Next, the compound (IV) so obtained is acylated with a carboxylic acid or a carboxylic acid derivative such as a carboxylic ester, carboxylic halide or carboxylic anhydride in a manner known *per se* in the art.

No particular limitation is imposed on the acyl group (W) as long as the hydroxyl group acylated with the acyl group is allowed to undergo allylic rearrangement. Examples of the acyl group include acetyl, benzoyl and formyl groups. Of these, acetyl group is particularly preferred.

This acylation reaction can be conducted in the presence of a base such as 4-dimethylaminopyridine, pyridine, triethylamine or N,N-dimethylaniline, usually under heat, for several hours to a few days, in some instances for several ten days, in an inert solvent, for example, an ether solvent such as tetrahydrofuran (THF) or diethyl ether, a chlorohydrocarbon solvent such as methylene chloride or chloroform, an aromatic hydrocarbon solvent such as benzene or toluene or an aprotonic solvent such as N,N-dimethylformamide (DMF), dimethylsulfoxide (DMSO) or acetonitrile. In this reaction, the carboxylic acid or its derivative can be used generally in an excess relative to the compound (IV), preferably in an amount 4-10 times the equivalent(s) of the latter. On the other hand, the base can be used generally in an amount about 8-20 times the equivalent(s) of the compound (IV).

The compound (V) obtained by the above acylation is then subjected to an allylic rearrangement reaction to provide the compound (VI) in which the 4-acyloxy group (WO) in the compound (V) has been rearranged to the 1-position.

This allylic rearrangement reaction can be conducted in an inert solvent, for example, an ether solvent such as THF or diethyl ether or an aromatic hydrocarbon solvent such as benzene or toluene while using, as a catalyst, at least one of Pd, Ni and Hg or a compound containing at least one of the above metals.

Preferred examples of the catalyst, namely, at least one of Pd, Ni and Hg or the compound containing at least one of the above metals are salts of divalent ions of these metals. Particularly preferred are palladium chloride, a dihalogenated palladium, and nickel dichloride, a dihalogenated nickel.

It is desirable for the allylic rearrangement reaction to use a suitable amount (for example, 0.01-0.5 equivalent) of a catalyst, preferably an appropriate amount (for example, 0.1-1 equivalent) of benzoquinone. The allylic rearrangement reaction can be conducted for about several hours under heat (for example, 60-100 ° C or so).

The compound (VI) obtained as described above is then converted to the compound (VII) by replacing the 1-acyloxy group with the electron-attracting eliminative group (X).

Examples of the electron-attracting eliminative group (X) include sulfonyloxy groups such as p-toluenesulfonyloxy, methanesulfonyloxy and trifluoromethanesulfonyloxy; and halogen atoms. Preferred are p-toluenesulfonyloxy, methanesulfonyloxy and trifluoromethanesulfonyloxy groups.

Among the compounds (VII), the compounds in which X is a sulfonyloxy group can each be obtained by deacylating the 1-acyl group (W) of the compound (VI) to form the compound represented by the formula (VI'):

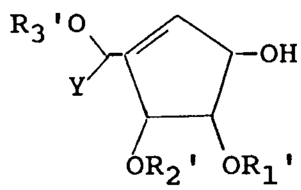

(VI')

and then causing a sulfonyl chloride such as p-toluenesulfonyl chloride, methanesulfonyl chloride or trifluoromethanesulfonyl chloride on the compound (VI').

The deacylation reaction can be conducted generally by treating the compound (VI) at 0-40 ° C or so, for 30 minutes to overnight, in the presence of about 2-5 equivalents of an inorganic base such as potassium carbonate, sodium hydrogencarbonate, sodium hydroxide or potassium hydroxide, in water or an alcohol solvent such as methanol or ethanol.

The reaction between the compound (VI') and the sulfonyl chloride can be conducted generally at 0-40 ° C or so, for about 1-3 days, in the presence of a base such as triethylamine, 4-dimethylaminopyridine or pyridine, in an ether solvent such as THF or diethyl ether, a chlorohydrocarbon solvent such as methylene chloride or chloroform, an aromatic hydrocarbon solvent such as benzene or toluene or an aprotonic solvent such as DMF, DMSO or acetonitrile.

On the other hand, the compound (VII) in which X is a halogen atom can be obtained by substituting the halogen atom for the hydroxyl group of the compound (VI') in accordance with a method known *per se* in the art, for example, in accordance with the method in which thionyl chloride is used or the method in which carbon tetrabromide and triphenylphosphine are used. As an alternative, an alkali halide (for example, NaI, LiI or KI) or the like may be reacted with the compound (VI).

The compound (VII) obtained as described above is finally reacted with the 3-deazaadenine derivative (VIII), whereby the target compound (I) of this invention can be obtained.

The above reaction is generally conducted by reacting, based on the compound (VII), about 2-5 equivalents of the 3-deazaadenine derivative (VIII) in the presence of 2-5 equivalents of a base and 1-2 equivalents of a crown ether at room temperature or under heat for 1 hour to 1 day or so in an inert solvent, for example, in an aprotonic solvent such as DMF, DMSO, N,N-dimethylacetamide (DMA), acetonitrile or THF. In this reaction, sodium hydride, potassium carbonate or the like can be used as the base while 15-crown-5, 18-crown-6 or the like can be employed as the crown ether.

Incidentally, the 3-deazaadenine derivative (VIII) can be prepared by the process described in the literature, Chem. Pharm. Bull. **12,** 866 (1964) or in a similar manner thereto. Although the 6-amino group of the compound may be protected, use of the compound without protection of the 6-amino group is preferred for conducting the reaction.

In the compound of this invention prepared as described above, the protecting groups of $R_{1'}$-$R_{3'}$ and the protecting group in $R_4$ can be eliminated by a desired deprotecting reaction.

This deprotecting reaction can be carried out by reacting the compound with a mineral acid such as hydrochloric acid or sulfuric acid, an organic acid such as trifluoroacetic acid, a mineral acid-organic acid mixture such as HBr/acetic acid or HBr/trifluoroacetic acid, or a boron compound such as $BBr_3$ or $BCl_3$ or subjecting the compound to catalytic hydrogenation in the presence of a catalyst such as palladium or rhodium in water, an alcohol solvent such as methanol, a chlorohydrocarbon solvent such as methylene

chloride, an aromatic hydrocarbon solvent such as benzene or an ether solvent such as THF.

Process 2:

The 6'-C-alkyl-3-deazaneplanocin A (I) of this invention can be prepared, for example, following the reaction scheme, namely, by protecting the 6-amino group and 2'- and 3'-hydroxyl groups of 3-deazaneplanocin A (X) to form the amino-protected derivative (XI), oxidizing the derivative into the 6'-formyl derivative (XII), alkylating the formyl derivative with an alkylating reagent, and if desired, protecting the 6'-hydroxyl group or removing these protecting groups.

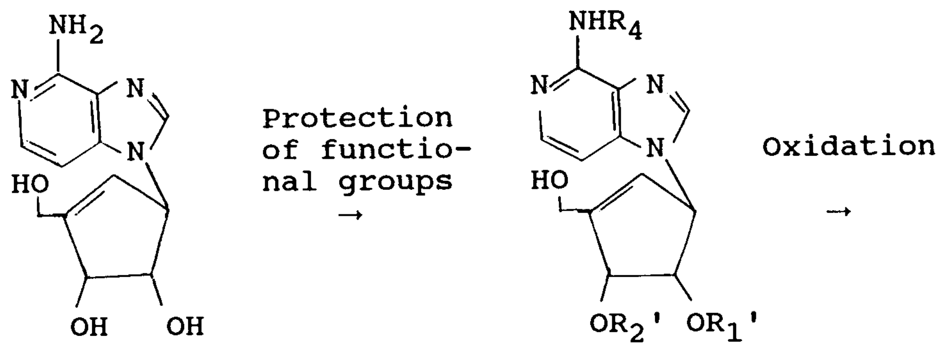


(X) (XI)

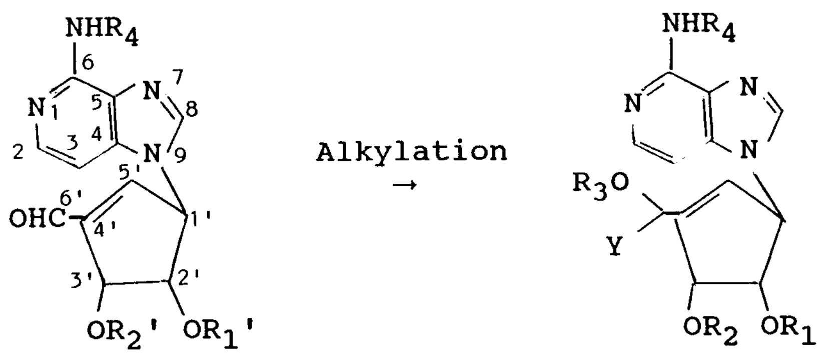


(XII) (I)

wherein $R_{1'}$ and $R_{2'}$ represent hydroxyl-protecting groups, $R_3$ means a hydrogen atom or a hydroxyl-protecting group, $R_4$ denotes a hydrogen atom or an amino-protecting group, and Y is a lower alkyl group.

In the above reaction scheme, no particular limitation is imposed on the protection of the amino group and hydroxyl groups of 3-deazaneplanocin A (X). Their protection can be effected usually by a method used in sugar or nucleic acid chemistry.

In particular, it is preferred to choose the amino-protecting group for $R_4$. Examples of preferred amino-protecting groups are those remaining stable under oxidizing and alkylating reaction conditions and permitting easy introduction and deprotection, including acyl-protecting groups such as benzoyl and phthaloyl groups and urethane-type protecting groups such as benzyloxycarbonyl and t-butoxycarbonyl groups.

Preferred hydroxyl-protecting groups include protecting groups capable of simultaneously protecting the 2'- and 3'-hydroxyl groups, such as isopropylidene, benzylidene and ethoxymethylene groups; and silyl protecting groups such as t-butyldimethylsilyl and diisopropylmethylsilyl groups, and ether-type protecting groups such as benzyl and methoxymethyl groups.

The oxidation of the amino-protected derivative (XI) can be effected in an inert solvent, using an

oxidizing agent such as an oxidizing agent of the chromic acid or manganic acid type. Namely, the oxidation reaction can be conducted at a temperature ranging from room temperature to the reflux temperature in a chlorohydrocarbon solvent such as methylene chloride, chloroform or dichloroethane, using an excess amount of an oxidizing agent such as $MnO_2$ or $BaMnO_4$.

The 6'-formyl derivative (XII) obtained by the above-described oxidation reaction is alkylated further and, if necessary, its 6'-hydroxyl group is protected or such protecting groups are removed, whereby the 6'-C-alkyl-3-deazaneplanocin A (I) according to this invention can be obtained.

The alkylating reaction can be effected by reacting the 6'-formyl derivative (XII) with an alkylating reagent, for example, a Grignard reagent such as methylmagnesium bromide or ethylmagnesium bromide, an alkyllithium such as methyllithium or ethyllithium, a trialkylaluminum such as trimethylaluminum or triethylaluminum, an organotitanium reagent such as trimethoxymethyltitanium and dichlorodimethyltitanium or an organozinc reagent such as dimethylzinc or diethylzinc in a chlorohydrocarbon solvent such as methylene chloride, an ether solvent such as THF or a hydrocarbon solvent such as hexane.

The protection of the 6'-hydroxyl group of the compound obtained by the alkylation of the 6'-formyl derivative (XII) can be conducted in a similar manner to the above-described case of $R_{3'}$. As an alternative, it can also be effected with an acyl group such as an acetyl or benzoyl group by a method employed in sugar or nucleic acid chemistry.

Further, the removal of the amino-protecting and hydroxyl-protecting groups form the compound obtained by the alkylation can be conducted generally by a method used in sugar or nucleic acid chemistry.

To collect the thus-prepared 6'-C-alkyl-3-deazaneplanocin A (I) from the reaction mixture, conventional separating and purification methods can be used. The compound (I) can be separated and purified by column chromatography, namely, by distilling off the solvent employed in the reaction, dissolving the residue in a solvent such as methanol, adsorbing the compound on an adsorbent such as silica gel and then eluting it with an elution solvent such as a chloroform-methanol solvent system.

The 6'-C-alkyl-3-deazaneplanocin A (I) can be converted to pharmaceutically-acceptable, non-toxic salts as needed, by a method known *per se* in the art.

Examples of such salts include inorganic acid salts such as the hydrochloride, sulfate and phosphate; and organic acid salts such as the acetate, propionate, tartrate, citrate, glycolate, gluconate, succinate, malate, glutamate, aspartate and methanesulfonate.

The 6'-C-alkyl-3-deazaneplanocin A (I) according to this invention contains some asymmetric carbons and hence includes isomers with respect to the asymmetric carbons. These isomers are all embraced by the present invention.

Incidentally, the compound (V) in Process 1 has two isomers with respect to the 6-position. The compound of this invention derived from one of these isomers, said isomer having a lower Rf in silica gel TLC, is preferred as its antiviral activity are superior. Further, it is generally preferable that, in the compound (VII), the group X at the 1-position is in the $\alpha$ configuration. The group X may, however, be either in the $\alpha$ configuration or in the $\beta$ configuration as long as the protecting group $R_{1'}$ for the second position is an acyl group.

To use the invention compound (I) or its salt as an antiviral agent, it is only necessary to administer an effective amount of the compound (I) or its salt as is or after formulating the same together with a known carrier into a dosage form.

Usable administration methods include oral administration or parenteral administration (for example, injection such as intravascular, intramuscular, subcutaneous or intraperitoneal administration; rectal administration; transocular administration; or the like). The above-mentioned formulation can be conducted depending on the administration method. Dosage forms include, for example, tablets, pills, powder, granules, capsules, injection, suppositories and eye drop. For their formulation, various carriers corresponding to the individual dosage forms can be used. For example, oral preparations such as tablets, granules and capsules can use excipients such as starch, lactose, sucrose, mannitol, carboxymethylcellulose, corn starch and inorganic salts; binders such as starch, dextrin, gum arabic, gelatin, hydroxypropylstarch, methylcellulose, sodium carboxymethylcellulose, hydroxypropylcellulose, crystalline cellulose, ethylcellulose, polyvinyl pyrrolidone and macrogol; disintegrators such as starch, hydroxypropylstarch, carboxymethylcellulose, sodium carboxymethylcellulose and hydroxypropylcellulose; surfactants such as sodium lauryl sulfate, soybean lecithin, sucrose fatty acid ester and Polysolvate 80; lubricants such as talc, wax, a hydrogenated vegetable oil, sucrose fatty acid ester, magnesium stearate and calcium stearate; fluidity promoters; corrigents; colorants; and perfumes.

The invention compound (I) or its salt can also be used in the form of a suspension, emulsion, syrup or elixir.

A parenteral preparation can generally use, as a diluent, a distilled water for injection, physiological saline, an aqueous glucose solution, a vegetable oil for injection, propylene glycol, polyethylene glycol or the like. Further, antiseptic, preservative and/or stabilizer can also be added as needed. From the safety standpoint, it is also possible to fill and lyophilize the parenteral preparation in a vial or the like and, immediately before use, to reconstitute it with a diluent. In addition, isotonicity, stabilizer, preservative, soothing agent and the like can also be incorporated suitably as needed.

The dose of the invention compound (I) or its salt varies depending on the administration route, the age, body weight and conditions of the patient, etc. In general, the daily dose may be about 5 mg to 1 g, preferably about 25-500 mg per adult in term of the compound (I). It is administered in 1-3 portions.

One of 6'-C-alkyl-3-deazaneplanocin As (I) which had been obtained as described above was tested for their pharmacological effects. The results will be described next.

(1) Antiviral activity:

By partly modifying the testing method reported in Antimicrobial Agents and Chemotherapy, **24**(3), 353-361 (1983), antiviral activity was analyzed in the following manner. 200 μℓ of Eagle's minimum essential medium (with 10% fetal calf serum), in which Vero cells (product of Flow General Inc., marketed by Dainippon Pharmaceutical Co., Ltd., available from National Institute of Health) were suspended at the concentration of 3 x $10^5$ cells/mℓ, were cultured in a 98-well flat bottom plate. Subsequent to dense monolayer culture, the medium was removed and 90 μℓ of Eagle's minimum essential medium (with 3% fetal calf serum) containing a test compound were added. The tissue culture infections dosis 50% ($TCID_{50}$) had been determined in advance without addition of any drug. The above-prepared culture plate was inoculated with 10 μℓ of a virus solution whose concentration was 100 times higher than $TCID_{50}$ determined in advance. The cells were cultured for 36 hours at 37°C under 5% $CO_2$. The cytopathic effect (CPE) was microscopically observed, whereby the minimum virus growth inhibitory concentration (MIC) was determined. The results are shown in Table 1.

(2) Measurement of cytotoxicity:

Added to 100 μℓ of Eagle's minimum essential medium were 100 μℓ of Eagle's minimum essential medium (with 10% fetal calf serum), in which Vero cells were suspended at the concentration of 5 x $10^4$ cells/mℓ as well as a test compound. The resultant mixture was cultured for 72 hours in a 98-well flat bottom plate. The cytotoxicity of the test compound was then measured by the MTT measuring method reported in J. Immunol. Methods, **65**, 55-63 (1983). The cytotoxicity is indicated in terms of the concentration ($ID_{50}$) which inhibited the growth of cells to 50%.

These results are summarized in Table 1.

Table 1

| Test compound | Minimum virus growth inhibitory concen. (μg/mℓ) | Minimum cytotoxicity concen. (μg/mℓ) | Chemotherapeutic index (MTC/MIC) |
|---|---|---|---|
| 6'-c-Methyl-3-deazaneplanocin A | 1.0 | 12.4 | 12.4 |
| 3-Deazaneplanosin A | 0.24 | 0.55 | 2.3 |
| Neplanocin A | 0.24 | 0.26 | 1.1 |

As is apparent from the results of the above-described tests on pharmacological activities, the 6'-C-alkyl-3-deazaneplanocin As (I) of the present invention show excellent antiviral activity and, compared to neplanocin A, their cytotoxicity are extremely low and their chemotherapeutic indices are great. As is also evident from the fact that no case of death was observed by 200 mg/kg oral administration of the above compound to mice, the 6'-C-alkyl-3-deazaneplanocin As (I) of the present invention have high safety and can hence be used effectively as antiviral agents.

The present invention will next be described in further detail by the following examples. It should, however, be borne in mind that the present invention is not limited at all by the following examples. In the following examples, "Merck Art. 9385 silica gel" (trade name) was used in silica gel flash columns.

Referential Example 1

Synthesis of [1-(methoxymethyloxy)ethyl]tributyltin

Diisopropylamine (32 mℓ, 230 mmol) was added to 400 mℓ of dry tetrahydrofuran (THF), followed by stirring at 0°C. The resultant mixture was added dropwise at the same temperature to 125 mℓ (200 mmol) of a 1.6 M butyllithium-hexane solution, followed by the dropwise addition of 52.8 mℓ (200 mmol) of tributyltin hydride at the same temperature upon an elapsed time of 5 minutes. Fifteen minutes later, the reaction mixture was cooled to -76°C, to which a solution of 11.2 mℓ (200 mmol) of acetaldehyde in 50 mℓ of dry THF was added dropwise. Thirty minutes later, 100 mℓ of a saturated aqueous ammonium chloride solution were added to the reaction mixture, and the reaction mixture was then poured into 250 mℓ of hexane. The hexane layer was washed twice with 500-mℓ portions of water. After dried over sodium sulfate, the hexane solution was filtered through a "Whatman Filter Paper Ips" (trade name, product of Whatman Company) and the resulting filtrate was concentrated under reduced pressure. The residue so obtained was dissolved in 50 mℓ of dry methylene chloride, to which 100 mℓ (790 mmol) of N,N-dimethylaniline were added. The resultant solution was stirred at 0°C, followed by the dropwise addition of 22.8 mℓ (300 mmol) of chloromethyl methyl ether. The temperature of the reaction mixture was allowed to rise to room temperature, at which the reaction mixture was stirred overnight. The reaction mixture was then poured into 2 ℓ of hexane. The organic layer was washed with chilled 0.5N-HCl (500 mℓ x 2), chilled water (500 mℓ x 1) and a chilled, saturated, aqueous sodium hydrogencarbonate solution (500 mℓ x 2). The organic layer was then filtered through a "Whatman Filter Paper Ips", followed by concentration under reduced pressure. The residue so obtained was purified by flash chromatography on a silica gel column [silica gel: 500 g; eluted with hexane → hexane-ethyl acetate (25:1)], whereby the title compound was obtained as a yellow syrup. Yield: 27.8 g (yield: 38%).

Example 1

Synthesis of (2S,3S,4S)-2,3-(isopropylidenedioxy)-4-[1-(methoxymethyloxy)ethyl]-4-hydroxy-5-cyclopentene

[1-(Methoxymethyloxy)ethyl]tributyltin (21.7 g, 60 mmol) prepared in Referential Example 1 was added to 240 mℓ of dry THF. After the resultant solution was cooled under stirring to -79°C, a solution of 1.6 M of n-butyllithium in 37.4 mℓ (60 mmol) of hexane was added dropwise to the solution. Twenty minutes later, a solution of 7.37 g (48 mmol) of (2S,3S)-2,3-(isopropylidenedioxy)-5-cyclopentene-4-one, which had been prepared in accordance with Tetrahedron Lett., **31,** 1509-1512 (1990), in 60 mℓ of dry THF was added dropwise at -78°C to the reaction mixture. After they were reacted for 2.5 hours at the same temperature, 2ℓ of chloroform were added to the reaction mixture, followed by the further addition of 400 mℓ of water. The resultant mixture was allowed to separate into layers. After the chloroform layer was filtered through a "Whatman Filter Paper Ips", the resulting filtrate was concentrated under reduced pressure. The residue so obtained was purified by flash chromatography on a silica gel column [silica gel: 400 g; eluted with hexane-acetone (25:1)], whereby the title compound was obtained as a clear syrup. Yield: 10.6 g (90%).
MASS, FAB (Pos.) m/e: 245 ($MH^+$).

Example 2

Synthesis of (2S,3S,4S)-2,3-(isopropylidenedioxy)-4-[1-(methoxymethyloxy)ethyl]-4-acetoxy-5-cyclopentene

(2S,3S,4S)-2,3-(Isopropylidenedioxy)-4-[1-(methoxymethyloxy)ethyl]-4-hydroxy-5-cyclopentene (5.93 g, 24.3 mmol) was added to 100 mℓ of methylene chloride. The resultant mixture was stirred at room temperature, to which 9.17 mℓ (97.2 mmol) of acetic anhydride, 2.97 g (24.3 mmol) of 4-dimethylaminopyridine (DMAP) and 13.55 mℓ (97.2 mmol) of triethylamine were added. After the mixture was stirred at room temperature for 10 days, the reaction mixture was added to 400 mℓ of chloroform, followed by washing with 80 mℓ of water. The organic layer was filtered through a "Whatman Filter Paper Ips" and the filtrate was concentrated under reduced pressure. The residue so obtained was purified by flash chromatography on a silica gel column [silica gel: 90 g; eluted with hexane-ethyl acetate (6:1)], whereby two types of diastereomers of the title compound, said diastereomers having different configurations at the 6-position, were obtained as syrups, respectively, including 3.7 g (yield: 53%) of a stereoisomer having a higher Rf value [Rf: 0.38; silica gel TLC plate "Art. 5715", product of Merck & Co., Inc.; hexane-ethyl acetate (3:1)] and 2.0 g (yield: 29%) of a stereoisomer having a lower Rf value [Rf: 0.32; hexane-ethyl acetate (3:1)]. Both the stereoisomers had the following mass spectrum.
MASS, CI (Pos.) m/e: 287 ($MH^+$).

Example 3

Synthesis of (1S,2S,3R)-1-acetoxy-2,3-(isopropylidenedioxy)-4-[1-(methoxymethyloxy)ethyl]-4-cyclopentene

Added to 50 mℓ of dry THF were 1.55 g (54.2 mmol) of (2S,3S,4S)-2,3-(isopropylidenedioxy)-4-[1-(methoxymethyloxy)ethyl]-4-acetoxy-5-cyclopentene [the compound having the lower Rf value (Rf = 0.32)] which was the compound obtained in Example 2. Further, 71 mg (0.27 mmol) of $PdCl_2(CH_3CN)_2$ and 235 mg (2.17 mmol) of 1,4-benzoquinone were added, followed by overnight reflux under an argon gas atmosphere. The reaction mixture was concentrated under reduced pressure. The residue so obtained was purified by flash chromatography on a silica gel column [silica gel: 100 g; eluted with hexane-ethyl acetate (5:1)], whereby 484 mg of the title compound were obtained as a syrup (yield: 31%). Further, 692 mg of the starting material were recovered (recovery rate: 45%).
MASS, FAB (Pos.) m/e: 287 ($MH^+$).

Example 4

Synthesis of (1S,2S,3R)-2,3-(isopropylidenedioxy)-4-[1-(methoxymethyloxy)ethyl]-1-hydroxy-4-cyclopentene

(1S,2S,3R)-1-Acetoxy-2,3-(isopropylidenedioxy)-4-[1-(methoxymethyloxy)ethyl]-4-cyclopentene (440 mg, 1.54 mmol) obtained above in Example 3 was dissolved in 10 mℓ of dry methanol, followed by the addition of 425 mg (3.08 mmol) of anhydrous potassium carbonate. The resulting mixture was stirred at room temperature for 5.5 hours. The reaction mixture was concentrated under reduced pressure and then dissolved in 100 mℓ of chloroform. After the chloroform solution was washed with an aqueous solution of sodium chloride, the chloroform layer was filtered through a "Whatman Filter Paper Ips" and the filtrate was concentrated under reduced pressure. The residue so obtained was purified by flash chromatography on a silica gel column (silica gel: 20 g; eluted with hexane-ethyl acetate (3:1)], whereby 346 mg of the title compound were obtained as a syrup (yield: 92%).
MASS, FAB (Pos.) m/e: 245 ($MH^+$).

Example 5

Synthesis of (1S,2S,3R)-1-[(p-toluenesulfonyl)oxy]-2,3-(isopropylidenedioxy)-4-[1-(methoxymethyloxy)ethyl]-4-cyclopentene

(1S,2S,3R)-2,3-(Isopropylidenedioxy)-4-[1-(methoxymethyloxy)ethyl]-1-hydroxy-4-cyclopentene (313 mg, 1.29 mmol) obtained above in Example 4 was stirred in 8 mℓ of dry methylene chloride, followed by the addition of 489 mg (2.57 mmol) of p-toluenesulfonyl chloride and 715 μℓ (5.14 mmol) of triethylamine. While shielding light, the resulting mixture was stirred for 2 days at room temperature. Water (20 mℓ) was added to the reaction mixture, followed by the addition of 50 mℓ of chloroform. The mixture thus formed was allowed to separate into layers. After the organic layer was filtered through a "Whatman Filter Paper Ips", the resultant filtrate was concentrated under reduced pressure. The residue so obtained was purified by flash chromatography on a silica gel column [silica gel: 40 g; eluted with hexane-ethyl aceate (5:1)], whereby 382 mg of the title compound were obtained as a syrup.
MASS, FAB (Pos.) m/e: 399 ($MH^+$).
$H^1$-NMR ($CDCl_3$) δ:
7.86(d), 7.33(d), 5.61(s), 5.20(m), 4.85(d), 4.72(t), 4.59(q), 4.46(q), 3.34(s), 2.45(s), 1.40-1.25(m).

Example 6

Synthesis of 2',3'-O-isopropylidene-6'-C-methyl-6'-O-(methoxymethyl)-3-deazaneplanocin A

3-Deazadenine (202 mg, 1.5 mmol) was added to 2 mℓ of dry DMF, followed by stirring. Then, 72.4 mg (1.5 mmol) of 50% NaH (in oil) were added at room temperature and 150 μℓ (0.75 mmol) of 15-crown-5 were added further. The resultant mixture was stirred for one hour. Added to the reaction mixture was a solution of 300 mg (0.75 mmol) of the tosylate obtained in Example 5. After the reaction system was purged with argon gas, the reaction mixture was stirred at 80 °C. Two hours later, the reaction mixture was cooled down to room temperature and was then concentrated under reduced pressure. Ethyl acetate (100 mℓ) was added to the residue so obtained, and insoluble matter was filtered off. The filtrate was washed with an

aqueous solution of sodium chloride. The ethyl acetate layer was filtered through a "Whatman Filter Paper Ips", and the filtrate was concentrated under reduced pressure. The residue so obtained was purified by flash chromatography on a silica gel column [Art. 9385: 40 g; eluted with $CHCl_3$-MeOH-conc. $NH_4OH$ (40:1:0.1)], whereby 150 mg of the title compound were obtained as crystals (yield: 55%).
MASS, FAB (Pos.) m/e: 361 ($MH^+$).

Example 7

Synthesis of 6'-C-Methyl-3-deazaneplanocin A

A protected derivative (150 mg, 0.42 mmol) of 2',3'-O-isopropylidene-6'-C-methyl-6'-O-(methoxymethyl)-3-deazaplanocin A obtained above in Example 6 was added to 5 mℓ of a 5M HCl-methanol solution. One hour later, the reaction mixture was concentrated. Several milliliters of methanol were added to the residue so obtained, followed again by concentration under reduced pressure. That procedure was repeated three times. Methanol was added to the residue so obtained. The methanol solution was adjusted to pH 10 with concentrated liquid ammonia, followed by concentration under reduced pressure. Ethanol was added, followed by concentration under reduced pressure. This procedure was repeated three times. The residue was dissolved in ethanol and then caused to be adsorbed on 500 mg of silica gel, followed by purification by flash chromatography on a silica gel column [silica gel: Art. 9385 - 10 g; eluted with $CHCl_3$-MeOH-concentrated $NH_4OH$ (65:25:2)], whereby 100 mg of the title compound were obtained as crystals (yield: 87%).

The compound was recrystallized from ethanol so that prism crystals were obtained:
MASS, FAB (Pos.) m/e: 277 ($MH^+$).
NMR ($CD_3OD$) $\delta$:
8.17(s), 7.65(d), 7.09(d), 5.96(s), 5.42(m), 4.62(d), 4.56(q), 4.16(t), 1.44(d).

**Claims**

**1.** A 6'-C-alkyl-3-deazaneplanocin A derivative represented by the following formula (I):

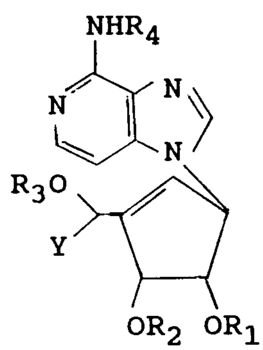

(I)

wherein Y represents a lower alkyl group, $R_1$, $R_2$ and $R_3$ individually mean a hydrogen atom or a hydroxyl-protecting group and $R_4$ denotes a hydrogen atom or an amino-protecting group, or a salt thereof.

**2.** A process for the preparation of a 6'-C-alkyl-3-deazaneplanocin A derivative of the following formula (I):

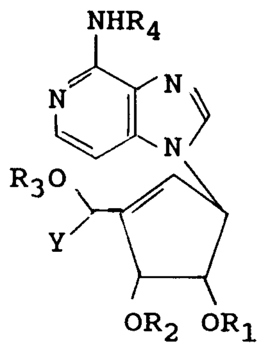

(I)

wherein Y represents a lower alkyl group, $R_1$, $R_2$ and $R_3$ individually mean a hydrogen atom or a hydroxyl-protecting group and $R_4$ denotes a hydrogen atom or an amino-protecting group, or a salt thereof, which comprises:

reacting in an inert solvent a cyclopentene derivative of the following formula (VII):

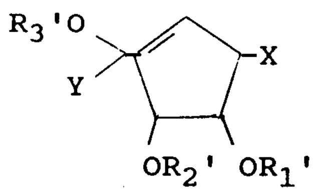

(VII)

wherein Y has the same meaning as defined above, $R_{1'}$, $R_{2'}$ and $R_{3'}$ individually mean a hydroxyl-protecting group and X denotes an electron-attracting eliminative group, with a 3-deazadenine derivative of the following formula (VIII):

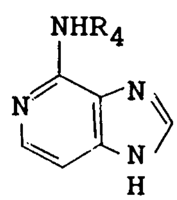

(VIII)

wherein $R_4$ has the same meaning as defined above; and, if desired,

removing one or more of the protecting groups from the reaction product.

3. The process of claim 2, wherein the cyclopentene derivative represented by the formula (VII) has been obtained by substituting or converting in an inert solvent a compound of the following formula (VI):

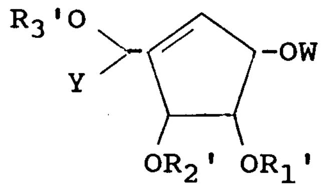

(VI)

wherein Y represents a lower alkyl group, $R_{1'}$, $R_{2'}$ and $R_{3'}$ individually mean a hydroxyl-protecting group and W denotes an acyl group.

4. The process of claim 3, wherein the compound of the formula (VI) has been obtained by subjecting a compound of the following formula (V):

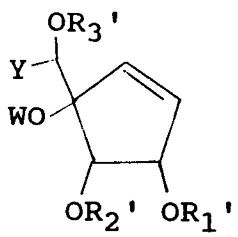

(V)

wherein Y represents a lower alkyl group, $R_{1'}$, $R_{2'}$ and $R_{3'}$ individually mean a hydroxyl-protecting group and W denotes an acyl group, to allylic rearrangement in an inert solvent in the presence of a catalyst selected from the group consisting of at least one of Pd, Ni and Hg and compounds comprising at least one of Pd, Ni and Hg.

5. The process of claim 4, wherein the compound of the formula (V) has been obtained by subjecting a compound of the following formula (IV):

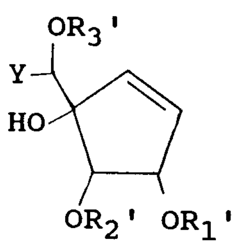

(IV)

wherein Y represents a lower alkyl group and $R_{1'}$, $R_{2'}$ and $R_{3'}$ individually mean a hydroxyl-protecting group, to acylation in an inert solvent.

6. The process of claim 5, wherein the compound of the formula (IV) has been obtained by reacting in an inert solvent a cyclopentenone derivative of the following formula (II):

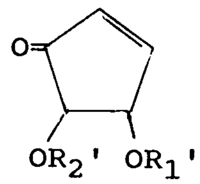

(II)

wherein $R_{1'}$ and $R_{2'}$ individually mean a hydroxyl-protecting group, with a compound of the following formula (III):

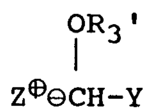

(III)

wherein Y represents a lower alkyl group, $R_{3'}$ means a hydroxyl-protecting group and Z denotes a counter-cation for stabilizing an alkyl anion.

**7.** An antiviral agent comprising as an active ingredient a 6'-C-alkyl-3-deazaneplanocin A derivative represented by the following formula (I):

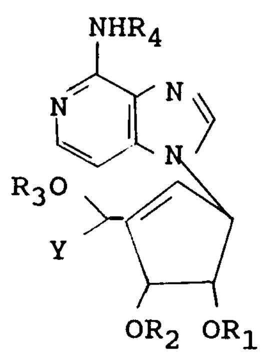

(I)

wherein Y represents a lower alkyl group, $R_{1'}$ $R_2$ and $R_3$ individually mean a hydrogen atom or a hydroxyl-protecting group and $R_4$ denotes a hydrogen atom or an amino-protecting group, or a salt thereof.

**8.** A cyclopentene derivative represented by the following formula (VII):

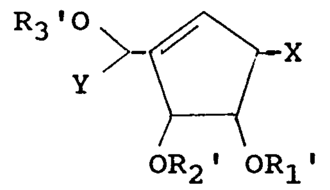

(VII)

wherein Y represents a lower alkyl group, $R_{1'}$, $R_{2'}$ and $R_{3'}$ individually mean a hydroxyl-protecting group and X denotes an electron-attracting eliminative group.

**9.** A process for the preparation of a cyclopentene derivative represented by the following formula (VII):

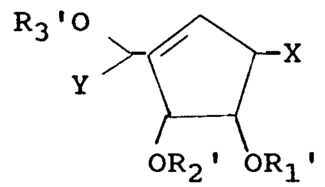

(VII)

wherein Y represents a lower alkyl group, $R_{1'}$, $R_{2'}$ and $R_{3'}$ individually mean a hydroxyl-protecting group and X denotes an electron-attracting eliminative group, which comprises substituting or converting in an inert solvent a compound represented by the following formula (VI):

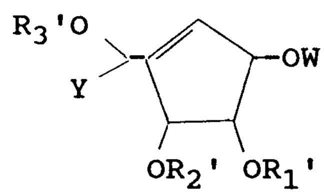

(VI)

wherein Y, $R_{1'}$, $R_{2'}$ and $R_{3'}$ have the same meanings as defined above and W denotes an acyl group.

**10.** The process of claim 9, wherein the compound represented by the formula (VI) has been obtained by subjecting a compound, which is represented by the following formula (V):

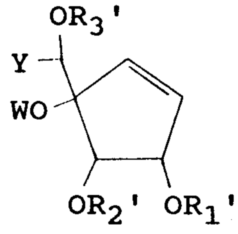

(V)

wherein Y represents a lower alkyl group, $R_{1'}$, $R_{2'}$ and $R_{3'}$ individually mean a hydroxyl-protecting group and W denotes an acyl group, to allylic rearrangement in an inert solvent in the presence of a catalyst selected from the group consisting of at least one of Pd, Ni and Hg and compounds comprising at least one of Pd, Ni and Hg.

**11.** The process of claim 10, wherein the compound represented by the formula (V) has been obtained by subjecting a compound of the following formula (IV):

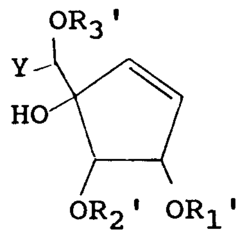

(IV)

wherein Y represents a lower alkyl group and $R_{1'}$, $R_{2'}$ and $R_{3'}$ individually mean a hydroxyl-protecting group, to acylation in an inert solvent.

**12.** The process of claim 11, wherein the compound represented by the formula (IV) has been obtained by reacting in an inert solvent a cyclopentenone derivative of the following formula (II):

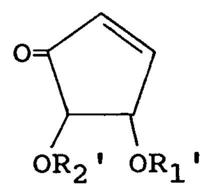

(II)

wherein $R_{1'}$ and $R_{2'}$ individually mean a hydroxyl-protecting group, with a compound of the following formula (III):

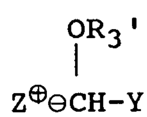

(III)

wherein Y represents a lower alkyl group, $R_{3'}$ means a hydroxyl-protecting group and Z denotes a counter-cation for stabilizing an alkyl anion.